# EUROPÄISCHE PATENTANMELDUNG

(11) **EP 2 218 395 A2**
(43) Veröffentlichungstag der Anmeldung: **18.08.2010**
(21) Anmeldenummer: 10152685.3
(22) Anmeldetag: 04.02.2010
(51) Int. Cl.: A61B 5/00, A61B 5/024

(54) **Vorrichtung und Verfahren zum Erfassen zumindest eines Vitalparameters einer Person; Vitalparametererfasssungssystem**

(30) Priorität: 12.02.2009 DE 102009008604
(71) Anmelder: Fraunhofer-Gesellschaft zur Förderung der Angewandten Forschung e.V., 80686 München (DE)
(72) Erfinder: Ciancitto, Fabio, 91056, Erlangen (DE); Tobola, Andreas, 91334, Hemhofen (DE); Hofmann, Christian, 90489, Nuernberg (DE); Couronné, Robert, 91058, Erlangen (DE)
(74) Vertreter: Hersina, Günter

(57) **Zusammenfassung**

Es wird eine Vorrichtung zum Erfassen zumindest eines Vitalparameters einer Person beschrieben, mit folgenden Merkmalen: einer optoelektronischen Sensoranordnung zum Erfassen des zumindest einen Vitalparameters mittels Lichttransmission oder Lichtremission, wobei die optoelektronische Sensoranordnung eine Lichtquelle (1) und ein lichtempfindliches Element (1') aufweist, wobei zum Erfassen des zumindest einen Vitalparameters mittels Transmission die Lichtquelle in einem ersten Seitenteil (110) eines Tragegestells (120) einer Brille angeordnet ist und das lichtempfindliche Element (1') in einem zweiten, dem ersten gegenüberliegenden Seitenteil (110') des Tragegestells (120) angeordnet ist, und wobei zum Erfassen des zumindest einen Vitalparameters mittels Lichtremission die Lichtquelle (1) und das lichtempfindliche Element (1') in dem selben Seitenteil (110) des Tragegestells (120) der Brille angeordnet sind.

## Beschreibung

Die vorliegende Anmeldung betrifft eine Messvorrichtung und ein Verfahren zum Überwachen, auch als "Monitoring" bezeichnet, eines oder mehrerer Vitalparameter eines Menschen, z. B. der Detektion eines arteriellen Plethysmogramms, der Herzrate, der Herzratenvariabilität, des Sauerstoffgehalts des arteriellen Blutes und der Pulswellenlaufzeit.

Bekannte Verfahren zum Erfassen von Vitalparametern sind beispielsweise die optische Plethysmographie und Pulsoximetrie. Dabei basieren die optische Plethysmographie und Pulsoximetrie auf ähnlichen Messverfahren. Diese bestehen aus einer aktiven Sensorvorrichtung, welche eine Lichtquelle und einen Photoempfänger enthält und so gestaltet ist, dass Licht die Gewebeschichten passiert und die verbleibende Lichtintensität vom Photoempfänger gemessen wird. Passiert das Licht die Gewebeschicht, erfährt es eine Dämpfung, die unter anderem abhängig von der Wellenlänge des Lichts, der Art und der Konzentration der Stoffe im durchstrahlten Gewebe und den Volumenänderungen des arteriellen Blutstromes ist. Der Photoempfänger wandelt das auftreffende Licht in einen Photostrom, dessen Amplitude von den durch Herzmuskelkontraktionen verursachten Volumenänderungen der arteriellen Gefäße moduliert wird.

Optische Pulsoximeter sowie optische Plethysmographen werden üblicherweise am Finger oder am Ohrläppchen des Patienten angebracht, weil dort die oberen Hautschichten sehr dicht mit arteriellen Blutgefäßen durchsetzt sind und der dämpfende Einfluss von Knochen- oder Fettgewebe minimal ist. Zum Einsatz kommen sowohl auf dem Transmissionsprinzip als auch auf dem Remissionsprinzip basierende Plethysmographen. Beim Remissionsverfahren wird der Finger nicht vollständig durchstrahlt wie beim Transmissionsverfahren, sondern es wird der nach der Lichteinstrahlung vom Gewebe emittierte, oder in anderen Worten reflektierte bzw. remittierte, Lichtanteil gemessen. Allen optischen Plethysmographen und Pulsoximetern für den Einsatz am Finger, angebracht beispielsweise durch einen Fingerclip an der Fingerkuppe, ist eine Einschränkung der Bewegungsfreiheit des Patienten gemein. Außerdem reagieren derartige Sensoren sehr empfindlich auf niedrige Durchblutung und/oder Vasokonstriktion der peripheren Arteriolen, was eine zuverlässige Aufnahme und Auswertung des Plethysmogramms und ableitbare Vitalparameter sowie Herzrate, Herzratenvariabilität, Sauerstoffgehalt des arteriellen Blutes und Pulswellenlaufzeit erheblich erschwert.

Bei anderen Verfahren wird die Messung durchgeführt, indem die Lichtquelle in ein Nasenloch des Patienten geführt wird und ein Lichtsensor in das andere Nasenloch eingeführt wird, um die Messung basierend auf dem Blutstrom in der Nasenscheidewand durchzuführen.

Der vorliegenden Anmeldung liegt die Aufgabe zugrunde, eine einfache bzw. effiziente Möglichkeit zu schaffen, einen oder mehrere Vitalparameter eines Menschen zu erfassen.

### Zusammenfassung

Ein Ausführungsbeispiel der vorliegenden Anmeldung schafft eine Vorrichtung zum Erfassen zumindest eines Vitalparameters einer Person mit folgenden Merkmalen: einer optoelektronischen Sensoranordnung zum Erfassen des zumindest einen Vitalparameters mittels Lichttransmission oder Lichtremission, wobei die optoelektronische Sensoranordnung eine Lichtquelle und ein lichtempfindliches Element aufweist, wobei für eine Erfassung des Vitalparameters mittels Lichttransmission die Lichtquelle in einem ersten Seitenteil eines Tragegestells einer Brille angeordnet ist und das lichtempfindliche Element in einem zweiten dem ersten gegenüberliegenden Seitenteil des Tragegestells angeordnet ist, so dass das Licht der Lichtquelle, wenn die Brille auf der Nase der Person aufgesetzt ist, die Nase zur Erfassung des Vitalparameters durchstrahlen kann, und für eine Erfassung des Vitalparameters mittels Lichtremission, die Lichtquelle und das lichtempfindliche Element in demselben Seitenteil des Tragegestells angeordnet sind, so dass, wenn die Brille auf der Nase der Person aufgesetzt ist, das lichtempfindliche Element einen durch die Nase reflektierten Anteil des von der Lichtquelle erzeugten Lichts empfangen kann.

Eine derartige Integration der optoelektronischen Sensoranordnung in eine Brille ermöglicht eine Messung des Vitalparameters basierend auf dem Blutfluss der Arteria dorsalis nasi und/oder der Arteria angularis und ist somit kaum von der Vasokonstriktion betroffen.

Vasokonstriktion ist der medizinische Fachbegriff für Gefäßverengung. Diese tritt beispielsweise unter Stresssituationen, Situationen niedrigen Blutdrucks oder Unterkühlung auf. Dabei werden die Gefäße der Beine und Arme verengt, um eine bessere Durchblutung bzw. einen höheren Blutdruck in den lebenswichtigen Organen, z. B. Gehirn und Herz, zu erreichen. Die Arteria dorsalis nasi und die Arteria angularis sind direkte Ableitungen der internen Halsschlagader und deshalb weniger von der Vasokonstriktion betroffen als die üblichen Messstellen wie beispielsweise Finger, Zeh oder Ohrläppchen. Ausführungsbeispiele ermöglichen somit auch unter den vorgenannten besonderen Situationen eine zuverlässige Erfassung der Vitalparameter.

Ein weiteres Ausführungsbeispiel der vorliegenden Erfindung für eine Transmissionsmessung zeichnet sich dadurch aus, dass das Tragegestell der Brille starr ist und die Lichtquelle und der Photosensor eine durch das Tragegestell definierte feste geometrische Anordnung zueinander aufweisen. Dadurch wird eine Verzerrung der Messergebnisse durch eine relative Bewegung der Lichtquelle gegenüber dem lichtempfindlichen Element verringert.

Gemäß einem weiteren Ausführungsbeispiel für ein Erfassen mittels Lichttransmission ist der Photosensor in dem einen Seitenteil des Tragegestells derart angeordnet, dass eine Richtung einer maximalen Lichtleistung des ersten Photosensors dem kürzesten Weg des Lichts von dem ersten Photosensor zu dem Lichtelement in dem gegenüberliegend angeordneten zweiten Seitenteil des Tragegestells entspricht. Dies ermöglicht eine Reduzierung des Energiebedarfs der Sensoranordnung bei gleicher Messqualität oder eine Erhöhung der Messqualität bei gleicher Energiezufuhr.

Bei einem weiteren Ausführungsbeispiel der vorliegenden Erfindung weist die optoelektronische Sensoranordnung mehrere jeweils in dem oder den Seitenteilen des Tragegestells räumlich verteilt angeordnete Lichtquellen und lichtempfindliche Elemente auf, die einander paarweise zugeordnet sind, und zusätzlich eine Steuereinrichtung, die ausgebildet ist, aus diesen mehreren Paaren von Lichtquellen und lichtempfindlichen Elementen dasjenige Paar auszuwählen, das im Vergleich zu den anderen Paaren eine höhere Messgüte oder Messqualität liefert. Damit kann dasjenige Messpaar ausgewählt werden, das im aufgesetzten Zustand einer Arterie am nächsten liegt, und somit die Güte und Zuverlässigkeit der Erfassung der Vitalparameter verbessert werden.

### Kurzbeschreibung der Figuren

Ausführungsbeispiele der vorliegenden Erfindung werden nachfolgend unter Bezugnahme auf die beiliegenden Zeichnungen näher erläutert.
- Fig. 1: zeigt eine schematische Darstellung einer Vorrichtung zum Erfassen zumindest eines Vitalparameters einer Person, wenn die Vorrichtung auf der Nase der Person aufgesetzt ist.
- Fig. 2: zeigt einen beispielhaften Verlauf eines optoelektronischen Messsignals erzeugt durch ein Ausführungsbeispiel einer Vorrichtung zum Erfassen zumindest eines Vitalparameters einer Person.
- Fig. 3: zeigt ein weiteres Ausführungsbeispiel einer Vorrichtung zum Erfassen zumindest eines Vitalparameters einer Person.
- Fig. 4: zeigt ein Ausführungsbeispiel einer Vorrichtung zum Erfassen zumindest eines Vitalparameters einer Person mittels Lichtremission.
- Fig. 5: zeigt eine schematische Darstellung einer Vorrichtung zum Erfassen zumindest eines Vitalparameters, bei dem die Vorrichtung in das Brillengestell integriert ist.
- Fig. 6: zeigt eine schematische Darstellung der wesentlichen in Frage kommenden Arterien für eine Messung von Vitalparametern.
- Fig. 7: zeigt eine schematische Darstellung eines Ausführungsbeispiels einer Vorrichtung zum Erfassen zumindest eines Vitalparameters mit einer Vielzahl von Lichtquellen und lichtempfindlichen Elementen zur örtlich selektiven Messung zur Bestimmung des zumindest einen Vitalparameters.
- Fig. 8: zeigt ein Flussdiagramm eines Verfahrens zum Erfassen zumindest eines Vitalparameters einer Person.
- Fig. 9: zeigt eine schematische Darstellung einer weiteren Anordnung einer Vielzahl von Lichtquellen und lichtempfindlichen Elementen.
- Fig. 10: zeigt eine schematische Darstellung einer in eine Brille integrierten Vorrichtung zum Erfassen zumindest eines Vitalparameters einer Person.
- Fig. 11: zeigt ein Blockschaltdiagramm eines Ausführungsbeispiels einer Steuer-/Auswerte-Elektronik.

Dabei werden in der vorliegenden Erfindung für Objekte und Funktionseinheiten, die gleiche oder ähnliche funktionelle Eigenschaften aufweisen, gleiche Bezugszeichen verwendet.

### Detaillierte Beschreibung

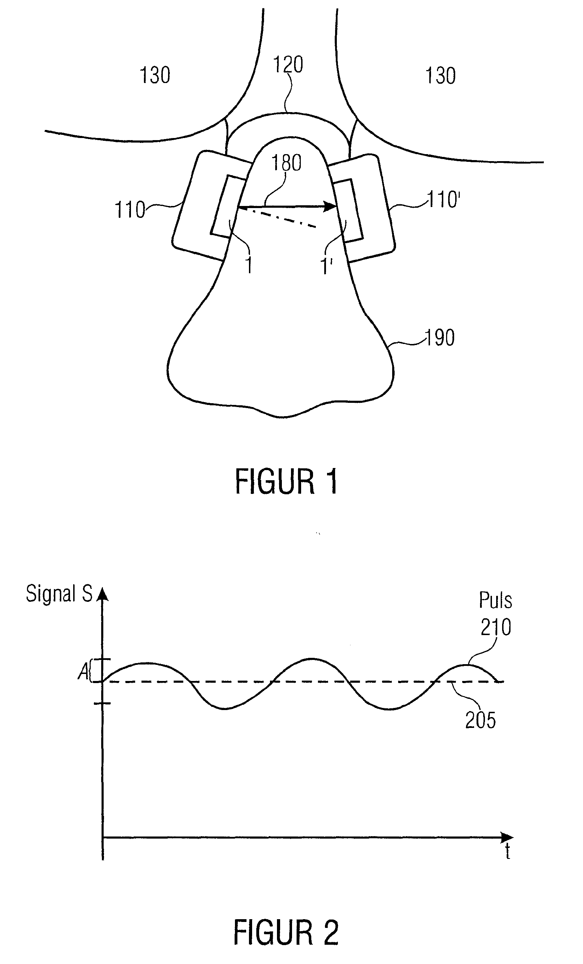

Fig. 1 zeigt eine schematische Darstellung der Vorrichtung zum Erfassen, in der das Tragegestell und die optoelektronische Sensoranordnung vergrößert dargestellt sind, um die Ausführungsbeispiele der vorliegenden Erfindung besser beschreiben zu können. Dabei zeigt Fig. 1 ein Ausführungsbeispiel einer Vorrichtung zum Erfassen zumindest eines Vitalparameters einer Person mit einer optoelektronischen Sensoranordnung zum Erfassen des zumindest einen Vitalparameters mittels Lichttransmission, wobei die optoelektronische Sensoranordnung eine Lichtquelle 1 und einen Lichtsensor 1' aufweist, und wobei die Lichtquelle 1 in einem ersten Seitenteil 110 eines Tragegestells 120 einer Brille angeordnet ist, das lichtempfindliche Element 1' in einem zweiten Seitenteil 110' des Tragegestells 120 angeordnet ist, und wobei der zweite Seitenteil 110' einen dem ersten Seitenteil 110 gegenüberliegenden Seitenteil des Tragegestells bildet. Fig. 1 zeigt die optoelektronische Sensoranordnung in einem auf die Nase der Person aufgesetzten Zustand. Durch die Integration der Lichtquelle 1 und des lichtempfindlichen Elements 1' in gegenüberliegende Seitenteile des Tragegestells 120 sind die Lichtquelle und das lichtempfindliche Element in einem auf die Nase aufgesetzten Zustand auf gegenüberliegenden Nasenseiten oder Nasenflügeln der Nase angeordnet. Gemäß Fig. 1 ist die Lichtquelle 1 auf der aus Sicht des Betrachters linken Nasenseite, oder in anderen Worten, in dem linken Seitenteil 110 des Tragegestells angeordnet, und das lichtempfindliche Element 1' auf der rechten Nasenseite, oder in anderen Worten in dem rechten Seitenteil 110' des Tragegestells 120, angeordnet. In Bezug auf den Träger der Brille sind die Seitenbezeichnungen links bzw. rechts entsprechend umgekehrt zu verwenden.

Ausführungsbeispiele der Vorrichtung weisen ferner eine Steuereinrichtung auf, die mit der Lichtquelle 1 und dem lichtempfindlichen Element 1' elektrisch verbunden ist (nicht gezeigt) und die ausgebildet ist, um für eine Messung eines Vitalparameters die Lichtquelle zu aktivieren, so dass diese ein Licht einer bestimmten Wellenlänge oder eines bestimmten Wellenlängenbereichs erzeugt, das durch die Nase 190 (siehe Pfeil 180) hindurch strahlt. Dabei wird ein Teil des Lichts durch die Gewebeschichten der Nase 190 absorbiert, so dass das lichtempfindliche Element 1' lediglich einen Teil des Lichts, nämlich den transmittierten Teil des Lichts, empfängt. Der Grad der Absorption bzw. Transmission hängt dabei von der Beschaffenheit des Gewebes und den Volumenänderungen des Blutstromes ab.

Fig. 2 zeigt einen beispielhaften schematischen Verlauf eines Messsignals, bei dem der statische Teil 205 (siehe gestrichelte Linie) den von der Dämpfung der Gewebeschichten abhängigen statischen Anteil des Signals (Bezugszeichen 205) und den diesem statischen Anteil überlagerten dynamischen bzw. variablen Anteil 210, der von den Volumenänderungen des arteriellen Blutstromes abhängig ist, und damit ein Maß für den Puls ist.

Wie aus Fig. 2 ersichtlich, kann mit einer Vorrichtung gemäß Fig. 1 ein arterielles Plethysmogramm erstellt werden, die Herz- bzw. Pulsrate, die Herzraten- bzw. Pulsvariabilität und die Pulsamplitude bestimmt werden. Zudem kann in Kombination mit einer weiteren Messung an einer anderen Stelle, z.B. dem Finger, oder in Kombination mit einem EKG (Elektrokardiogramm) die Pulswellenlaufzeit bestimmt werden.

Die Vitalparameter können beispielsweise aus der Amplitude und dem Amplitudenverlauf des elektrischen Signals, z.B. dem Verlauf eines Photostroms oder einer Photospannung, ermittelt werden, oder aus einer Fourier-Transformation dieses elektrischen Signals. Dabei weisen Ausführungsbeispiele der vorliegenden Erfindung eine Steuer- oder Treiberschaltung auf, die die Lichtquelle 1 mit hoher Lichtintensität aber sehr kurzen Pulsen ansteuert, um den Stromverbrauch der Lichtquelle gering zu halten und auf der Empfängerseite, nämlich des lichtempfindlichen Elements 1', einen Spitzen-Detektor, auch als Peak-Detektor bezeichnet, um eine zuverlässige digitale Abtastung des analogen plethismographischen Signals trotz der kurzen Lichtimpulse zu ermöglichen.

Weitere Möglichkeiten der optoelektronischen Messung basieren an Stelle der Messung der Amplitude auf der Fourier-Transformation des gemessenen Signals.

Für die Erfassung des Sauerstoffgehalts (Oximetrie) weist ein Ausführungsbeispiel der Vorrichtung zum Erfassen zwei Lichtquellen auf, die jeweils ein Licht in einem voneinander unterschiedlichen Lichtwellenbereich erzeugen. Durch die unterschiedliche Färbung des mit Sauerstoff gesättigten Hämoglobins erfahren die zwei durchstrahlenden Lichter eine unterschiedliche Absorption, die das lichtempfindliche Element misst. Eine Auswerteeinheit kann dann beispielsweise mittels eines Vergleichs der Messergebnisse mit einer Referenztabelle die Sauerstoffsättigung des Blutes in den Arterien bestimmen.

Beispiele für Lichtquellen sind Leuchtdioden, im Englischen auch als Light Emitting Diodes (LED) bezeichnet, und für die lichtempfindlichen Elemente, so genannte Photodioden. Dabei kann ein Ausführungsbeispiel, das den Sauerstoffgehalt des Blutes messen soll, eine rote Diode, die beispielsweise ein sichtbares Licht im 660-Nanometer-Bereich erzeugt, und eine infrarote Leuchtdiode als weitere Lichtquelle aufweisen, die beispielsweise ein Licht im für den Menschen nicht-sichtbaren Wellenlängenbereich von 940 Nanometer erzeugt.

Allgemein ausgedrückt, ist die Lichtquelle ausgebildet, ein Licht einer ersten Wellenlänge oder eines ersten Wellenlängenbereichs zu erzeugen, und die zweite Lichtquelle ausgebildet, ein Licht einer zweiten Wellenlänge bzw. eines zweiten Wellenlängenbereichs zu erzeugen, wobei die erste und die zweite Wellenlänge respektive der erste und der zweite Wellenlängenbereich unterschiedlich sind.

Fig. 1 zeigt ferner die Gläser 130 der Brille, die über das Tragegestell 120 miteinander mechanisch verbunden sind und mittels des ersten Seitenteils 110 und des zweiten Seitenteils 110' des Tragegestells auf der Nase 190 der Person aufliegen.

Dabei kann das Tragegestell der Brille flexibel in dem Sinne ausgestaltet sein, dass der Abstand des ersten Seitenteils 110 und des zweiten Seitenteils 110' des Tragegestells sich leicht verändert, sich beispielsweise beim Aufsetzen auf eine Nase 190 abhängig von der Nasenbreite vergrößert, oder im Wesentlichen starr sein, und sich somit der Abstand des ersten Seitenteils 110 und des zweiten Seitenteils 110' und damit auch der Lichtquelle 1 und des lichtempfindlichen Elements 1' zueinander im Wesentlichen nicht verändert.

Die starre Ausführung des Tragegestells bewirkt, dass die Lichtquelle 1 und das lichtempfindliche Element 1' eine durch das Tragegestell definierte feste geometrische Anordnung zueinander aufweisen, und somit die Gefahr, dass der Gleichanteil 205 des Signals durch Änderung des Abstands der Lichtquelle zu dem lichtempfindlichen Element geändert wird, und somit die Messergebnisse verfälscht.

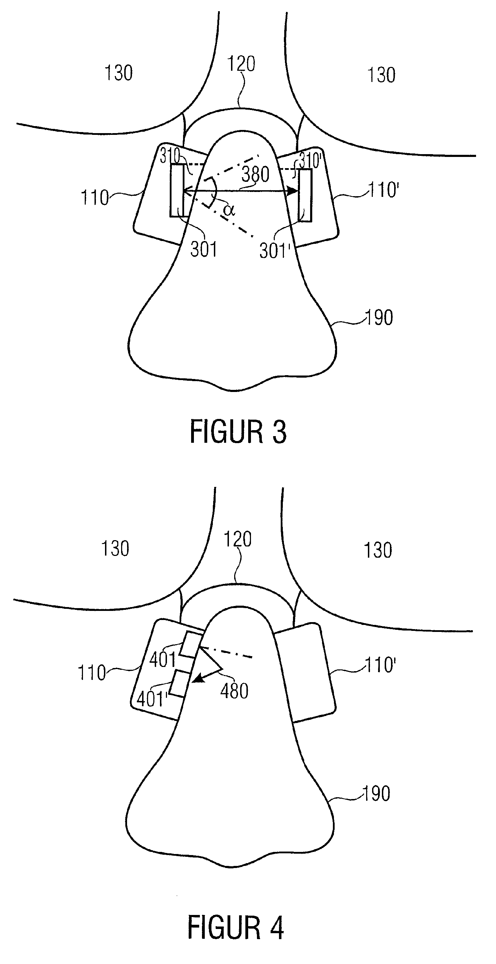

Fig. 3 zeigt ein weiteres Ausführungsbeispiel, ähnlich zu dem in Fig. 1, bei dem jedoch abweichend zu Fig. 1 die Lichtquelle 301 derart in dem ersten Seitenteil 110 des Tragegestells 120 angeordnet ist, dass eine Richtung einer maximalen Lichtleistung der Lichtquelle einen kürzesten Weg des Lichts (siehe Bezugszeichen 380) von der Lichtquelle 301 zu dem lichtempfindlichen Element 301' entspricht. Die Richtung der maximalen Lichtleistung kann auch als Hauptemissionsrichtung bezeichnet werden.

Ausführungsbeispiele gemäß Fig. 3 weisen beispielsweise Lichtquellen auf, die Licht in einem Öffnungswinkel α abgeben, wobei diese in der Mitte des Öffnungswinkels, die auch als Lot oder Lotrichtung der Lichtquelle bezeichnet werden kann (siehe ebenfalls Pfeil mit Bezugszeichen 380), typischerweise eine maximale Leistung abgibt, und mit zunehmender Abweichung von diesem Lot bzw. dieser Hauptstrahlrichtung weniger Leistung abgibt. Diese Eigenschaft kann der Lichtquelle immanent innewohnen oder durch entsprechende Bündelung, z. B. durch Linsenstrukturen im Verkappungsmaterial einer Leuchtdiode, erreicht werden. Eine Lichtquelle mit einer derartigen fokussierten Lichtabstrahlung ermöglicht eine Reduzierung des Energiebedarfs, bei qualitativ gleichwertigen Messergebnissen im Vergleich zu breiter abstrahlenden bzw. Leuchtquellen mit größerem Öffnungswinkel α, oder andersherum, ermöglicht bei ähnlichem Energiebedarf eine Verbesserung der Signalamplitude und damit auch der Signalgüte oder Signalqualität.

Entsprechend sind lichtempfindliche Elemente, deren Effizienzgrad bei der Umsetzung des empfangenen Lichts in elektrische Energie richtungsabhängig ist, ebenfalls so angeordnet, dass das von der Lichtquelle 301 erzeugte Licht aus dieser Richtung empfangen wird (siehe Pfeil mit dem Bezugszeichen 380). Fig. 3 zeigt weiter einen transparenten Bereich 310 in dem ersten Seitenteil 110 des Tragegestells, der so angeordnet ist, dass das Licht der Lichtquelle 301 im Wesentlichen ungehindert in Richtung Nase austreten kann. Entsprechend kann auch der zweite Seitenteil 110' einen in dem zumindest verwendeten Lichtwellenbereich transparenten Bereich 310' aufweisen, so dass das lichtempfindliche Element 301' das Licht 380 im Wesentlichen ungedämpft empfangen kann.

Entsprechende transparente Bereiche 310, 310' können auch in Ausführungsbeispielen gemäß Fig. 1 angewendet werden.

Fig. 4 zeigt ein Ausführungsbeispiel einer Vorrichtung zum Erfassen zumindest eines Parameters einer Person mit einer optoelektronischen Sensoranordnung zum Erfassen des zumindest einen Vitalparameters mittels Lichtremission, wobei die optoelektronische Sensoranordnung eine Lichtquelle 401 und ein lichtempfindliches Element 401' aufweist, und wobei die Lichtquelle 401 und das lichtempfindliche Element 401' im demselben Seitenteil des Tragegestells 120 einer Brille angeordnet sind, oder in anderen Worten, in einem auf die Nase aufgesetzten Zustand der Brille allgemein auf derselben Seite (hier der ersten bzw. linken Seite aus Sicht des Betrachters) der Nase angeordnet sind. Die Lichtquelle 401 und das lichtempfindliche Element 401' sind beispielsweise in einer Ebene und nahe zueinander angeordnet. Die Lichtquelle 401 strahlt in das Gewebe der Nase, wobei ein Teil des Lichts absorbiert wird, ein Teil des Lichts typischerweise transmittiert und ein anderer Teil in dem Gewebe bzw. in der Nase allgemein reflektiert wird. Das lichtempfindliche Element empfängt den reflektierten remittierten Anteil des Lichts der Lichtquelle 401 (wie dies durch den Fall 480 symbolisch dargestellt ist). Dabei sind die Lichtquelle 401 und das lichtempfindliche Element 401' räumlich so zueinander angeordnet, dass ein möglichst großer Anteil des reflektierten Lichts der Lichtquelle empfangen werden kann oder ein möglichst großer Anteil des Lichts der Lichtquelle reflektiert wird und von dem lichtempfindlichen Element 401' empfangen werden kann.

In alternativen Ausführungsbeispielen können die Lichtquelle 401 und das lichtempfindliche Element 401' auch auf der anderen Seite der Nase, beispielsweise in dem zweiten Seitenteil 110' des Tragegestells 120 angeordnet sein, oder an einer anderen Stelle, z.B. auf dem Nasenrücken, indem das Tragegestell ein zusätzliches Aufliegeelement dort aufweist oder nicht zwei diskrete Seitenteile 110 und 110' aufweist, sondern ein durchgehendes Element, das sich von der einen Seite der Nase über den Nasenrücken auf die andere Seite der Nase erstreckt.

In diesem Zusammenhang wird auch darauf hingewiesen, dass weitere Ausführungsbeispiele der Vorrichtung zum Erfassen zumindest eines Vitalparameters eine optoelektronische Sensoranordnung zum Erfassen des zumindest einen Vitalparameters aufweisen können, die ausgeführt ist, diesen mittels Lichttransmission und Lichtremission zu ermitteln, und dies gleichzeitig oder alternierend bzw. jeweils auswählbar. In anderen Worten können die Ausführungsbeispiele gemäß Fig. 1, 3 und 4 miteinander kombiniert werden.

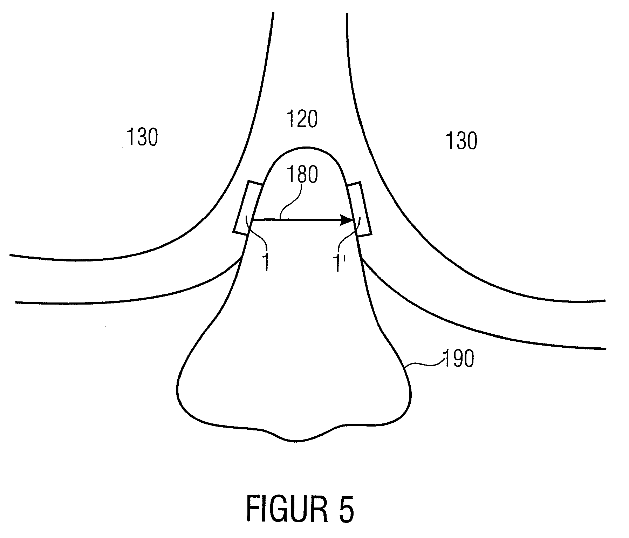

Fig. 5 zeigt ein Ausführungsbeispiel einer Vorrichtung zum Erfassen zumindest eines Vitalparameters, die in eine Brille integriert ist, die im Gegensatz zu den Ausführungsbeispielen gemäß Fig. 1, 3 und 4 kein separates Tragegestell 120 aufweist, sondern bei der das Brillengestell selbst auch das Tragegestell 120 ist. Dabei zeigt Fig. 5 das entsprechende Ausführungsbeispiel gemäß Fig. 1, wobei weitere Ausführungsbeispiele beispielsweise auch eine optoelektronische Sensoranordnung gemäß Fig. 3 oder 4 oder eine der später noch näher beschriebenen aufweisen kann.

Bei Vergleichsmessungen hat sich herausgestellt, dass die Amplitude A (siehe Fig. 2) des aufmodulierten Pulssignals 210 desto größer ist, je größer der Einfluss der Arterien auf die Lichttransmission und Lichtremission ist. Dieser Einfluss ist am größten für den Fall, dass der Lichtstrahl durch die Arterie transmittiert und/oder in ihr reflektiert wird, oder in anderen Worten, ist umso größer, je geringer der Abstand zwischen der Arterie und dem Lichtstrahlverlauf 180, 380 oder 480 ist, und nimmt entsprechend mit zunehmendem Abstand von diesem Lichtstrahlverlauf 180, 380 und 480 ab.

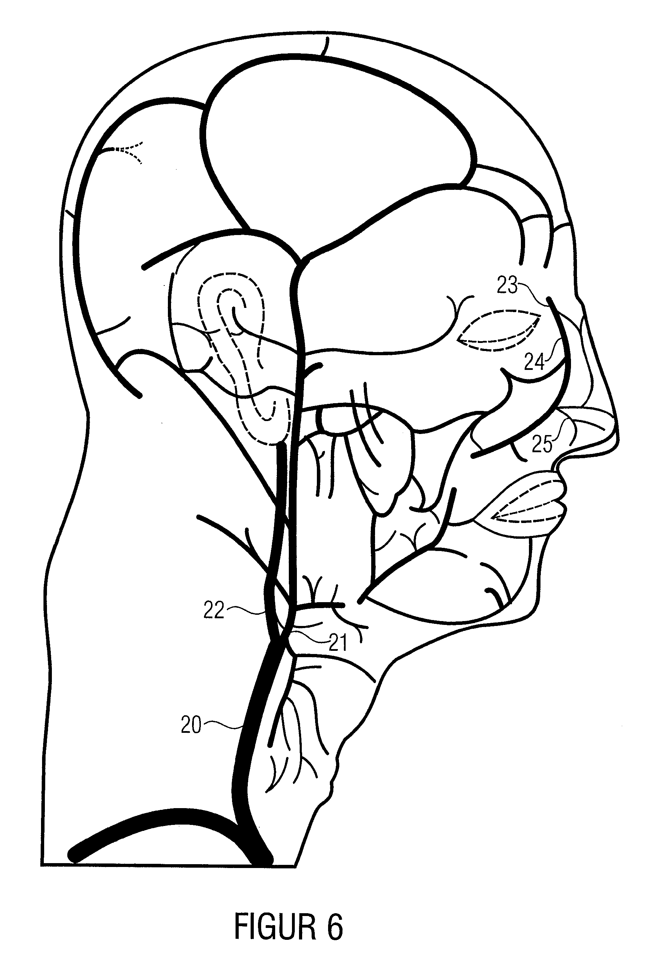

Fig. 6 zeigt die Lage der Arteria carotis communis 20, der Arteria carotis externa 21, der Arteria carotis interna 22, der Arteria dorsalis nasi 23, der Arteria angularis 24 und der Arteria lateralis nasi 25.

Ausführungsbeispiele der Vorrichtung zum Erfassen zumindest eines Vitalparameters messen die Vitalparameters anhand der Arteria dorsalis nasi und/oder der Arteria angularis (bei Lichttransmission) und einer Abzweigung derselben (für die Reflexionsmessung). Obwohl die Arteria dorsalis nasi 23 und die Arteria angularis 24 typischerweise bei allen Menschen einen im Wesentlichen ähnlichen Verlauf und eine ähnliche Position in Bezug auf die Nase haben, ist jedoch die Form der Nase selbst, beispielsweise in Bezug auf ihre Höhe und Breite, von Mensch zu Mensch sehr unterschiedlich. Damit kann eine bestimmte räumliche Anordnung der Lichtquelle 1 und des Lichtsensors 1' in einem Seitenteil (Reflexionsmessung) oder in beiden Seitenteilen (Transmissionsmessung) des Tragegestells 120 für eine Messung des Vitalparameters optimal oder zumindest gut geeignet sein, während aufgrund einer unterschiedlichen Nasenform die selbe Anordnung der Lichtquelle 1 und des lichtempfindlichen Elements 1' in dem einen Seitenteil 110 und/oder dem zweiten Seitenteil 110' schlechtere Ergebnisse oder sogar unbrauchbare Ergebnisse liefern.

Ausführungsbeispiele können daher eine personenspezifische, d.h. auf die Position und den Verlauf der Arterien speziell angepasste, Position und Anordnung der Lichtquelle 1 und des lichtempfindlichen Elements 1' in dem oder den Seitenteil(en) des Tragegestells 120 aufweisen, um jeweils eine optimale Messung zu ermöglichen.

Darüber hinaus kann jedoch die Brille während des Tragens verrutschen und/oder beim Ab- und Wiederaufsetzen an verschiedenen Positionen der Nase aufgesetzt werden, so dass selbst bei einer derartigen personenspezifischen Anpassung der Position und Anordnung der Lichtquelle und des lichtempfindlichen Elements, es dadurch zu Verschlechterungen in der Qualität und Güte der Messergebnisse kommen kann.

Weitere Ausführungsbeispiele der Vorrichtung zum Erfassen zumindest eines Vitalparameters einer Person weisen eine Vielzahl von Lichtquellen und eine Vielzahl von lichtempfindlichen Elementen auf, die beispielsweise jeweils paarweise einander zugeordnet sind, so dass eine Lichtquelle und ein lichtempfindliches Element ein Messpaar ergeben, mittels dessen ein Signal S (siehe Fig. 2) für die Ermittlung eines Vitalparameters gemessen werden kann.

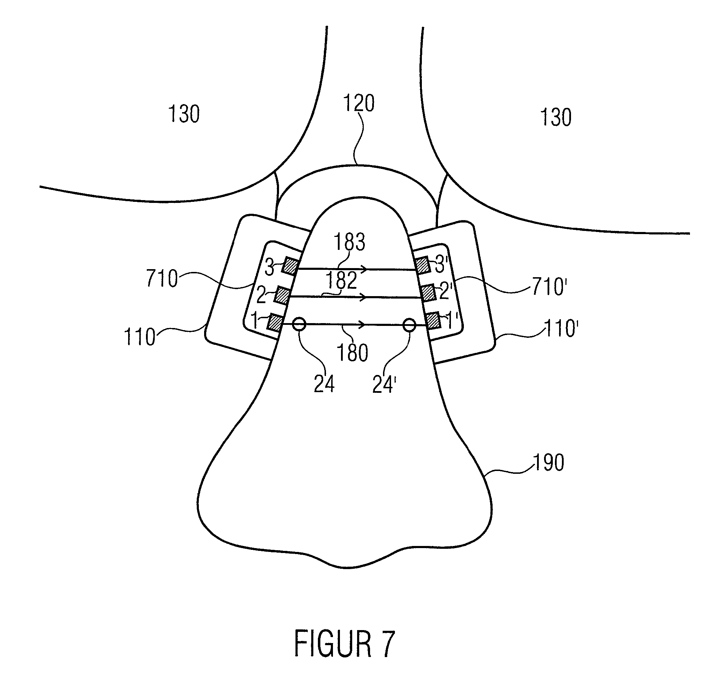

Fig. 7 zeigt ein Ausführungsbeispiel der Vorrichtung, die eine erste Lichtquelle 1, eine zweite Lichtquelle 2 und eine dritte Lichtquelle 3 (siehe schraffierte Flächen) in dem ersten Seitenteil 110 des Trägergestells 120 der Brille aufweist, und ein erstes lichtempfindliches Element 1' (siehe schraffierte Flächen), ein zweites lichtempfindliches Element 2' und ein drittes lichtempfindliches Element 3', die in dem zweiten Seitenteil 110' des Trägergestells 120 der Brille angeordnet sind. Die erste, zweite und dritte Lichtquelle können dabei in einem Bauteil 710 integriert sein, das wiederum in dem ersten Seitenteil 110 integriert ist, und das erste, zweite und dritte lichtempfindliche Element können in einem Bauteil 710' integriert sein, das wiederum in dem zweiten Seitenteil 110' des Trägergestells 120 integriert sein kann, wie dies in Fig. 7 gezeigt ist. Fig. 7 zeigt ein Ausführungsbeispiel, bei dem die zweite Lichtquelle 2 räumlich über der ersten Lichtquelle 1 in dem Bauelement 710 bzw. dem ersten Seitenteil 110 angeordnet ist, und die dritte Lichtquelle 3 wiederum räumlich über der zweiten Lichtquelle 2 in dem Bauelement 710 bzw. in dem ersten Seitenteil 110 angeordnet ist. Entsprechend ist das zweite lichtempfindliche Element 2' räumlich über dem ersten lichtempfindlichen Element 1' und das dritte lichtempfindliche Element 3' räumlich über dem zweiten lichtempfindlichen Element 2' in dem Bauelement 710' bzw. dem zweiten Seitenteil 110' angeordnet. Ferner ist in dem Ausführungsbeispiel gemäß Fig. 7 die erste Lichtquelle 1 und das erste lichtempfindliche Element 1' in Bezug auf ihre Position zum Tragegestell im Wesentlichen auf der selben Höhe angeordnet, die zweite Lichtquelle 2 auf der selben Höhe wie das lichtempfindliche Element 2' und die dritte Lichtquelle 3 auf der selben oder im Wesentlichen auf der selben Höhe wie das dritte lichtempfindliche Element 3'. Ferner bilden die erste Lichtquelle 1 und das erste lichtempfindliche Element 1' ein erstes Messpaar bzw. sind einander für eine Messung zum Erfassen des zumindest einen Vitalparameters zugeordnet, die zweite Lichtquelle 2 mit dem zweiten lichtempfindlichen Element 2' ein zweites Messpaar und die dritte Lichtquelle 3 mit dem dritten lichtempfindlichen Element 3' ein drittes Messpaar. Die Vorrichtung zum Erfassen des zumindest einen Vitalparameters weist ferner eine Steuereinrichtung (in Fig. 7 nicht gezeigt) auf, die wahlweise eine Messung für die Erfassung des zumindest einen Vitalparameters mittels des ersten Messpaars, des zweiten Messpaars oder des dritten Messpaars durchführen kann, wobei die Steuereinrichtung ausgerüstet ist, die erste Lichtquelle 1 zu aktivieren oder so anzusteuern, dass die erste Lichtquelle ein oder mehrere Lichtpulse einer bestimmten Wellenlänge oder in einem bestimmten Wellenlängenbereich zu erzeugt, und das erste lichtempfindliche Element 1' auszuwählen, um dessen Signal S auswerten oder an eine spezielle Auswerteeinrichtung auszulesen.

Fig. 7 zeigt die Vorrichtung zum Erfassen in einem auf der Nase 190 aufgesetzten Zustand, wobei Fig. 7 die Arteria angularis 24 zeigt.

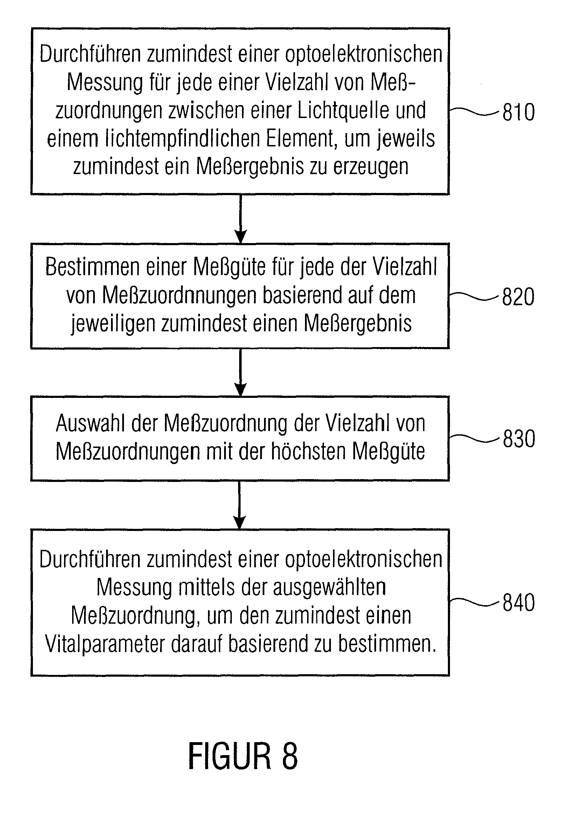

Im Folgenden wird anhand der Fig. 7 und der Fig. 8 ein Ausführungsbeispiel eines Verfahrens zum Erfassen zumindest eines Vitalparameters einer Person mittels einer optoelektronischen Sensoranordnung (wie beispielsweise in Fig. 7 dargestellt). Dabei ist der zu bestimmende Vitalparameter der Person ein Pulsparameter, z.B. eine Pulsrate, die basierend auf dem abgetasteten Amplitudenverlauf der Pulskurve 210, wie sie in Fig. 2 dargestellt ist, bestimmt wird. Dabei wird die Amplitude des Signals gleichzeitig als Maß für eine Qualität bzw. Güte des optoelektronischen Messsignals herangezogen.

Allgemein formuliert wird in einem ersten Schritt des Verfahrens ein bestimmtes Messpaar der drei Messpaare basierend auf der Qualität oder Güte einer oder mehrerer optoelektronischer Messungen, die auch als Testmessungen bezeichnet werden können, ausgewählt, um das ausgewählte Messpaar dann in einem nächsten Schritt für die eigentliche Bestimmung oder Erfassung des Vitalparameters einzusetzen.

Fig. 8 zeigt ein Flussdiagramm eines Ausführungsbeispiels eines Verfahrens zum Bestimmen eines Vitalparameters einer Person. Dieses Verfahren kann beispielsweise durch eine Steuervorrichtung der Vorrichtung zum Erfassen des zumindest einen Vitalparameter oder eines Vitalparametererfassungssystems durchgeführt werden.

Schritt 810 umfasst das Durchführen zumindest einer optoelektronischen Messung in demselben Wellenlängenbereich oder derselben Wellenlänge für jede einer Vielzahl von Messzuordnungen zwischen einer Lichtquelle einer Vielzahl (hier 3) von Lichtquellen und einem einer Vielzahl (hier 3) von lichtempfindlichen Elementen, um jeweils zumindest ein amplitudenabhängiges Messergebnis zu erzeugen.

In Schritt 820 wird basierend auf den jeweiligen zumindest einem Messergebnissen für jede der drei bzw. Vielzahl von Messzuordnungen eine Messgüte bestimmt.

In Schritt 830 wird dann die Messzuordnung der Vielzahl von Messzuordnungen mit der Höchstmessgüte ausgewählt, um dann in Schritt 840 zumindest eine optoelektronische Messung mittels der ausgewählten Messzuordnung durchzuführen, um den zumindest einen Vitalparameter darauf basierend zu bestimmen.

Auf die Vorrichtung gemäß Fig. 7 bezogen, wird zunächst für die erste Messzuordnung die erste Lichtquelle 1 dem ersten lichtempfindlichen Element 1' zugeordnet, eine ausreichende Anzahl von optoelektronischen Messungen durchgeführt, um ein Minimum und ein Maximum während des Pulsverlaufs 210 und somit die Amplitude des Signals 210 zu bestimmen. Dasselbe wird für die zweite Messzuordnung aus der zweiten Lichtquelle 2 und dem zweiten lichtempfindlichen Element 2' sowie der dritten Messzuordnung aus der dritten Lichtquelle 3 und dem dritten lichtempfindlichen Element 3' durchgeführt. Es wird beispielsweise jeweils ein Minimumwert und ein Maximumwert des Signals 210 bestimmt und daraus die Amplitude A abgeleitet. Die Amplitude A dient gleichzeitig als Gütemaß in dem Sinne, dass je höher die Amplitude ist, desto höher die Güte des Signals ist. Dieses Gütemaß kann daher auch als amplitudenabhängiges Gütemaß bezeichnet werden. Wie in Fig. 7 zu sehen ist, geht der Lichtstrahl 180 für die optoelektronische Messung der ersten Messzuordnung durch die Arteria angularis und weist somit die höchste Amplitude auf. Der Lichtstrahl 182 der zweiten Messzuordnung geht zwar nicht mehr durch die Arteria angularis, ist jedoch immer noch näher an dieser als der Lichtstrahl 183 (Lichtpfad bzw. Pfad des Lichtstrahls 183) der dritten Messzuordnung, wird daher stärker vom Puls der Arteria angularis 24 beeinflusst und weist somit eine geringere Amplitude als das Messsignal der ersten Messzuordnung auf, αber eine höhere Amplitude als das Messsignal der dritten Messzuordnung.

Wie zuvor anhand von Fig. 8 dargelegt, wird in Schritt 820 die Messgüte, hier die Amplitude, bestimmt und in Schritt 830 die erste Messzuordnung 1-1' ausgewählt, da sie die höchste Messgüte aufweist. In Schritt 840 wird dann die optoelektronische Messung oder die mehreren optoelektronischen Messungen durchgeführt, um den oder die Vitalparameter der Person zu bestimmen. Die anderen Messzuordnungen 2-2' und 3-3' werden nicht oder zunächst nicht verwendet.

Durch ein Abnehmen und Wiederaufsetzen der Brille oder ein Verschieben der Brille auf dem Nasenrücken kann sich die relative Position der ausgewählten ersten Messzuordnung 1-1' gegenüber der Arteria angularis verändern und eine Verschlechterung der Messergebnisse bewirken.

Weitere Ausführungsbeispiele des vorliegenden Verfahrens sind daher ausgebildet, die Güte der Messsignale der aktuellen oder aktiven Messzuordnung 1-1' zu überwachen, beispielsweise mit einem Schwellwert zu vergleichen. Sinkt die Güte der Messungen der aktiven oder momentan ausgewählten Messzuordnung 1-1' unter diesen Schwellwert, können beispielsweise wieder die Schritte 810 bis 830 durchgeführt werden, um zu prüfen, ob eine andere Messzuordnung jetzt eine bessere Messgüte liefern kann, um dann wieder mit Schritt 840 die Bestimmung der Vitalparameter durchzuführen.

In einem weiteren Ausführungsbeispiel wird nach Schritt 820 nicht nur eine Messzuordnung für die folgenden tatsächlichen Messungen ausgewählt, sondern die verschiedenen Messzuordnungen entsprechend ihrer Güte in einer Rangfolge gespeichert, und statt einer erneuten Durchführung der Schritte 810 bis 830 bei Unterschreiten des Güteschwellwerts durch die aktive Messzuordnung die gemäß der Rangfolge nächste bzw. nächst beste Messzuordnung als aktive Messzuordnung für die folgenden Messungen zur Bestimmung des Vitalparameters verwendet. In dem Fall von Fig. 7 wird der Messzuordnung 1-1' Rang 1, die Messzuordnung 2-2' Rang 2 und der dritten Messzuordnung 3-3' Rang 3 zugeordnet. Entsprechend würde gemäß dem Ausführungsbeispiel des Verfahrens bei Unterschreiten des Güteschwellwerts durch die erste Messzuordnung als nächstes die zweite Messzuordnung 2-2'aktiviert. Wenn auch diese den Güteschwellwert unterschreitet, würde dann die dritte Messzuordnung 3-3' aktiviert, und erst wenn diese auch den Güteschwellwert unterschreitet, die Schritte 810 und 820 sowie die Bestimmung der Rangfolge durchgeführt.

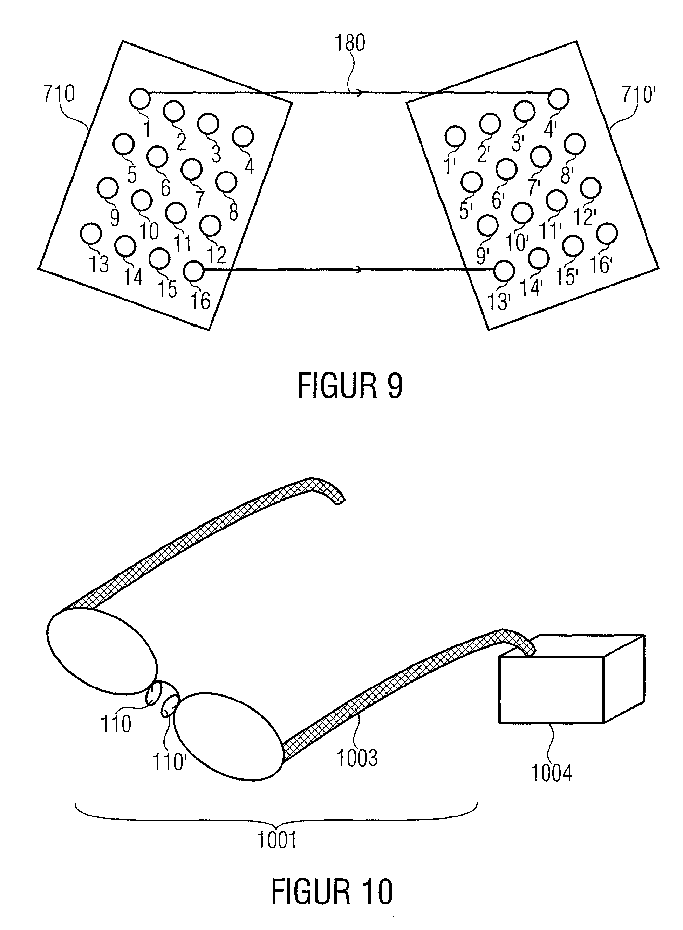

Fig. 9 zeigt ein Ausführungsbeispiel eines Bauelements 710 mit m=16 Lichtquellen 1 bis 16 und einem zweiten Bauelement 710' mit ebenfalls n=16 lichtempfindlichen Elementen 1' bis 16'. Gemäß einem Ausführungsbeispiel des Verfahrens werden die Leuchtquellen 1 bis 16 jeweils ihrer gegenüberliegend angeordneten entsprechenden lichtempfindlichen Element 1' bis 16' zugeordnet, z.B. bilden Lichtquelle 1 und lichtempfindliches Element 1 eine erste Zuordnung 1-1', Lichtquelle 2 mit lichtempfindlichem Element 2' eine zweite Messzuordnung, Lichtquelle 3 mit dem lichtempfindlichen Element 3 eine dritte Zuordnung, usw. Dabei sind für das Transmissionsverfahren die Lichtquellen und die ihnen zugeordneten lichtempfindlichen Elemente gegenüberliegend angeordnet bzw. so angeordnet, dass sie einen möglichst großen Querschnittsbereich der Nase für die Messung erfassen können. Anders ausgedrückt, die jeweils einander zugeordneten Lichtquellen und lichtempfindlichen Elemente sind so angeordnet, dass sie symmetrisch zueinander angeordnet sind.

Ausführungsbeispiele, die eine erste Anzahl m von Lichtquellen aufweisen, die in einer Reihe räumlich zueinander angeordnet sind, und die einer entsprechenden Reihe von lichtempfindlichen Elementen auf der gegenüberliegenden Seite aufweisen, ermöglichen eine Kompensation einer Verschiebung der Brille in eben diese Richtung. In dem Ausführungsbeispiel gemäß Fig. 7 könnte als eine Verschiebung der Brille in vertikaler Richtung zumindest teilweise ausgeglichen werden.

Eine flächige Anordnung von einer ersten Anzahl von Lichtquellen, wie sie beispielsweise Fig. 9 zeigt, ermöglicht eine Kompensation von Verschiebungen der Brille in vertikaler und horizontaler Richtung gegenüber den Arterien, die in der Nase verlaufen.

Basierend auf Fig. 9 wird ein weiteres Ausführungsbeispiel beschrieben. Dabei werden die Schritte 810 und 820 wie zuvor anhand von Fig. 8 beschrieben durchgeführt. In diesem Ausführungsbeispiel wird jedoch nicht nur die Messzuordnung mit der höchsten Messgüte ausgewählt, sondern zumindest ein Teil, z.B. die besten 8, ausgewählt und in einer Rangfolge gemäß ihrer Messgüte gespeichert. Während der Durchführung der optoelektronischen Messungen mit der ausgewählten Messzuordnung, basierend auf denen der Vitalparameter bestimmt wird, werden diese gleichzeitig zum kontinuierlichen Überwachen der Güte der momentan ausgewählten Messzuordnung verwendet. Fällt die amplitudenabhängige Messgüte der momentan ausgewählten Messzuordnung unter einen Messschwellwert, wird gemäß der Rangfolge die Messzuordnung mit der nächst höchsten Messgüte ausgewählt und die Messungen mit dieser fortgeführt, um den Vitalparameter zu erfassen. Entsprechend wird verfahren, wenn auch die Messgüte der nächsten Messzuordnung unter den Messschwellwert fällt, usw. Ist auch die Messgüte der achten Messzuordnung kleiner als der Messschwellwert, wird erneut eine optoelektronische Messung für alle 16 Messzuordnungen durchgeführt, wie anhand von Schritt 810 und folgende beschrieben.

In Ausführungsbeispielen können die Lichtquellen und lichtempfindlichen Elemente in Matrizen angeordnet sein, z.B. jeweils in einer 4x4 Matrix wie in Fig. 9, oder in beliebigen anderen flächenmäßigen Anordnungen, sie können eine beliebige Anzahl von Lichtquellen m mit m=1,2,3,... und eine beliebige Anzahl von lichtempfindlichen Elementen n=1,2,3... aufweisen. Dabei kann die Anzahl m der Lichtquellen gleich der Anzahl n der lichtempfindlichen Elemente sein, oder größer oder kleiner. Beispielsweise können Ausführungsbeispiele eine Lichtquelle 1 und eine Vielzahl von lichtempfindlichen Elementen, z.B. 1' bis 16', aufweisen, wobei sich die Vielzahl von Messzuordnungen dann aus verschiedenen Zuordnungen der einen Lichtquelle zu verschiedenen lichtempfindlichen Elementen ergibt. Andere Ausführungsbeispiele können entsprechend umgekehrt, eine Anzahl n von Lichtquellen aufweisen und lediglich ein lichtempfindliches Element. Ferner kann dasselbe lichtempfindliche Element mehreren Lichtquellen zugeordnet sein, oder in anderen Worten, mehrere Messzuordnungen dieselben lichtempfindlichen Elemente aufweisen, oder umgekehrt. Darüber hinaus kann eine Steuereinrichtung ausgebildet sein, Messungen für alle möglichen oder einen Teil aller möglichen Zuordnungen durchzuführen, um variabel die besten Messzuordnungen zu bestimmen. Dabei sind die Messzuordnungen nicht auf die 1-zu-1 Zuordnungen begrenzt, so wie sie in den Fig. 7 und 9 dargestellt sind, sondern können beliebige andere Zuordnungen umfassen, beispielsweise eine Messzuordnung zwischen der Lichtquelle 1 und dem lichtempfindlichen Elemente 16' und zwischen der Lichtquelle 6 und dem lichtempfindlichen Element 7'. Alternativ können die möglichen oder auswählbaren Messzuordnungen vorgegeben sein, und die Steuerung wählt lediglich aus diesen die besten Messzuordnungen aus.

In Bezug auf ihre flächenmäßige Anordnung und Verteilung werden die Lichtquellen und lichtempfindlichen Elemente insbesondere an den Stellen des ersten Seitenteils und zweiten Seitenteils des Trägergestells angeordnet, für die bei normalem Sitz der Brille auf der Nase damit zu rechnen ist, dass zumindest einer der Lichtpfade (z.B. 180, etc.) der verschiedenen Messzuordnungen durch die Arterie hindurchgeht, oder zumindest einen möglichst geringen Abstand von ihr hat, um eine möglichst hohe Güte für die Messung der Vitalparameter zu erreichen.

In weiteren Ausführungsbeispielen der Vorrichtung zum Erfassen können die Lichtquellen und lichtempfindlichen Elemente ohne Bauelemente 710, 710' direkt in den ersten Seitenteil 110 oder zweiten Seitenteil 110' integriert sein.

Dies gilt entsprechend für Ausführungsbeispiele, die auf der Messung mittels Lichtremission basieren.

Weitere Ausführungsbeispiele können ausgebildet sein, auch den Sauerstoffgehalt des Blutes zu messen. Diese weisen dann Lichtquellen auf, die in einem anderen Lichtwellenbereich betrieben werden können, und die beispielsweise neben oder zwischen Lichtquellen, wie sie in den Fig. 7 und 9 dargestellt sind, angeordnet werden. In diesem Falle werden als Messzuordnungen Mess-Tripel aus zwei Lichtquellen mit verschiedenen Lichtwellenlängen und einem lichtempfindlichen Element definiert. Auch in diesen Ausführungsbeispielen können Ausführungsbeispiele der beschriebenen Verfahren angewendet werden, z.B. ein aktives Mess-Tripel für die eigentlichen Messungen ausgewählt werden, und auf andere Mess-Tripel zurückgegriffen werden, wenn die Qualität des aktiven Mess-Tripels unter einen gewissen Qualitätsschwellwert fällt.

In anderen Worten, in Ausführungsbeispielen der Vorrichtung zum Erfassen kann eine Vielzahl von Lichtquellen eine erste Untermenge von Lichtquellen aufweisen, die ausgebildet sind, um ein Licht eines ersten Wellenlängenbereichs zu erzeugen, und eine zweite Untermenge von Lichtquellen aufweisen, die ausgebildet sind, um ein Licht eines zweiten von dem ersten unterschiedlichen Wellenlängenbereich zu erzeugen.

Dabei wird der Begriff Messzuordnung allgemein für die Zuordnung zwischen Lichtquellen und lichtempfindlichen Elementen verwendet. Dabei bezeichnet der Begriff Messpaar den besonderen Fall, dass eine Lichtquelle einem lichtempfindlichen Element zugeordnet wird, z.B. für eine optische Plethysmographie, und der Begriff Mess-Tripel den besonderen Fall, dass einem lichtempfindlichen Element zwei Lichtquellen, die mit unterschiedlichen Wellenlängen betrieben werden zugeordnet werden, z.B. für die optische Oximetrie.

Fig. 10 zeigt eine schematische Darstellung eines Ausführungsbeispiels einer Messvorrichtung, das in einen ersten Seitenteil 110 des Tragegestells und einem zweiten Seitenteil 110' des Tragegestells einer Brille 1100 integriert ist. Fig. 10 zeigt ferner eine Steuer- und Auswerteeinheit 1004, die die Steuer- und Auswerteelektronik aufweist und beispielsweise eine Batterie oder einen Akkumulator zur Stromversorgung aufweist. Die Steuereinheit 1004 kann über Kabel mit dem oder den Lichtquellen und lichtempfindlichen Elementen elektrisch verbunden sein und/oder durch in die Brillenbügel 1003 und allgemein in die Brille 1001 integrierte Leiterbahnen. Die Steuereinrichtung 1004 kann ferner ein Funkmodul aufweisen, z.B. Bluetooth-basiert, um die Vitalparameter oder Statistiken über Vitalparameter zu übertragen oder anderen Informationen, z.B. Ansteuerprogramme oder -Parameter zu empfangen.

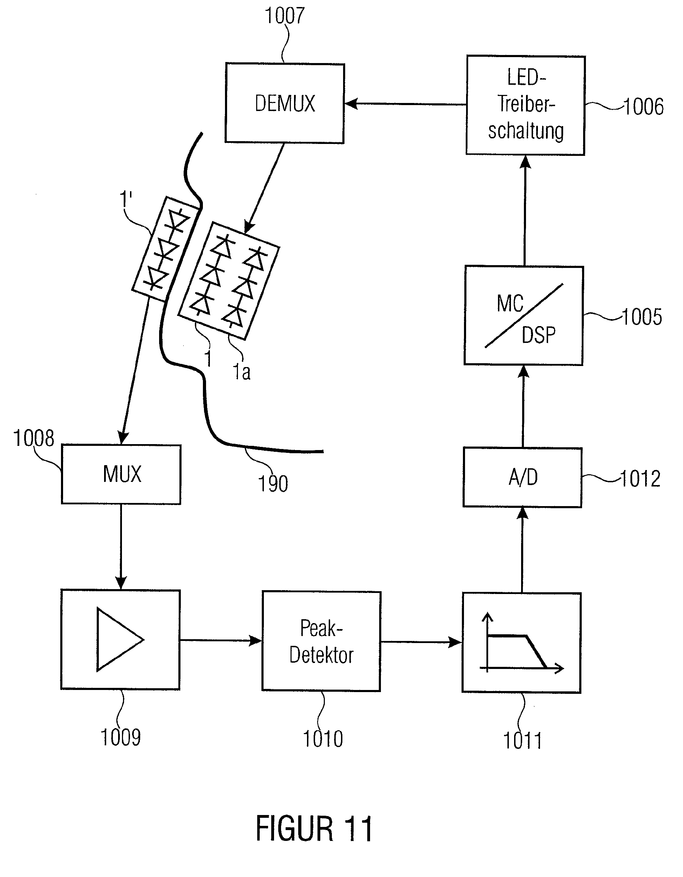

Fig. 11 zeigt ein Blockdiagramm für eine Vorrichtung zum Erfassen eines Vitalparameters, die in eine Brille 1001 (siehe Fig. 10) integriert ist, in der eine oder mehrere Lichtquellen unterschiedlicher Wellenlänge, z.B. 1 und 1a, eine oder mehrere Lichtempfänger, z.B. 1', und eine Ansteuer-/Auswerteelektronikeinheit 1004 integriert sind. Fig. 11 zeigt beispielhaft eine Lichtquelle 1, die ausgebildet ist, ein Licht in einer ersten Wellenlänge zu erzeugen, eine weitere Lichtquelle 1a, die ausgebildet ist, Licht in einer zweiten von der ersten unterschiedlichen Wellenlänge zu erzeugen, beide symbolisch durch eine Serienschaltung von Leuchtdioden dargestellt, und ein lichtempfindliches Element 1' auf der anderen Seite der Nase, symbolisch durch eine Serienschaltung von Photodioden dargestellt. Die Lichtquellen und die Lichtempfängerdioden werden direkt seitlich auf dem Nasenrücken angebracht und die Elektronikeinheit 1004 kann beispielsweise davon räumlich getrennt und durch Kabel verbunden an einer weniger störenden Körperstelle, z.B. am Rücken, der Brust einem Stirnband oder Hut oder direkt an dem Bügel 1003 in einem Bereich hinter dem Ohr angebracht werden. Die Ansteuer- und Auswerteelektronikeinheit 1004 weist beispielsweise einen Mikrocontroller (µc) und/digitalen Signalprozessor (DSP) 1005 auf, einen LED-Treiberschaltung 1006, einen Demultiplexer 1007 im Falle eines Ausführungsbeispiels, bei dem verschiedenen Diodenzuordnungen, wie zuvor erläutert, ausgewählt werden können, einen analogen Verstärker 1009, einen SpitzenDetektor bzw. Peak-Detektor 1010, einen analogen Filter 1011 und einen Analog-Digital-Wandler (A/D-Wandler) 1012 und ggf. einen Multiplexer 1008.

In Ausführungsbeispielen kann ein geeigneter Mikrocontroller, vorzugsweise ein digitaler Signalprozessor 1005 die Ansteuerung der einzelnen Komponenten der Anordnung sowie die Aufzeichnung, Verarbeitung und Auswertung der sich aus der Anordnung ergebenden Signalverläufe übernehmen. Der Mikrocontroller bzw. digitale Signalprozessor steuert die LED-Treiberschaltung 1006, ggf. den Demultiplexer 1007, übernimmt die Verteilung der erzeugten Signale auf die einzelnen Lichtquellen 1, 1a und die anhand von Fig. 8 beschriebene Auswahl von Messzuordnungen in Ausführungsbeispielen, die eine Ortsanpassung der Messung ermöglichen.

Nach dem Analog-Digital-Wandler 12 wird das digitalisierte Signal vom Mikrocontroller bzw. von dem digitalen Signalprozessor 1005 empfangen und verarbeitet. Anschließend wird das Signal mit einer Schaltung 1009 verstärkt, mit einem Peak-Detektor 1010 wird der Puls zeitlich verlängert, um eine verbesserte Abtastung zu ermöglichen. Am Ende wird das Signal mit einer Schaltung 1011 gefiltert. Danach werden die plethysmographischen Signale, dies ist abhängig von der Anzahl der Wellenlängen, von dem Mikrocontroller bzw. dem digitalen Signalprozessor 1005 weiterverarbeitet und Vitalparameter aus diesem Signal oder diesen Signalen oder in Kombination mit anderen physiologischen Parametern berechnet. Dabei weisen Ausführungsbeispiele des Messsystems bzw. der Vorrichtung zum Erfassen des zumindest einen Vitalparameters eine Treiberschaltung 1006 auf, die die Lichtquellen bzw. Leuchtdioden mit hoher Lichtintensität bzw. Strom treiben kann, und den Betrieb der Lichtquellen 1, 1a mit sehr kurzen Impulsen durchführen kann, um einen hohen Stromverbrauch des Messsystems zu vermeiden, und die einen Peak-Detektor 1010 aufweisen, um eine zuverlässige digitale Abtastung des analogen plethysmographischen Signals zu ermöglichen.

In Anbetracht der vorhergehenden Erläuterungen liegt eine Aufgabe verschiedener Ausführungsbeispiele der vorliegenden Erfindung darin, die Erfassung einer Pulswellenkurve und davon abgeleitet der Blutsauerstoffsättigung an einem bislang nicht nutzbaren Ort, der Nase, nicht invasiv und wenig beeinträchtigend zu realisieren. Dabei ermöglichen Ausführungsbeispiele der Erfindung das Tragen der Messeinrichtung nicht nur im klinischen Umfeld, sondern auch im häuslichen bzw. mobilen Umfeld, welches mit bekannten Fingerclip-Pulsoximetern nicht möglich ist.

Dabei werden Ausführungsbeispiele der Sensorvorrichtung wie eine normale Brille getragen, so dass die Einschränkung der Bewegungsfreiheit des Patienten auf ein Minimum reduziert werden kann. Der Finger ist nicht blockiert und der Patient nicht in seiner Bewegungsfreiheit eingeschränkt. Die bei herkömmlichen Pulsoximetern beobachtete beschränkte oder verfälschte Auswertung wichtiger Vitalparameter, z.B. Herrate, Herzratenvariabilität, Sauerstoffgehalt des arteriellen Blutes und der Pulswellenlaufzeit, welche aufgrund niedriger Durchblutung bzw. Vasokonstriktion der peripheren Gefäße beruht, ist am Ableitort Nase wesentlich reduziert. Der physiologische Grund dafür ist, dass die peripheren Gefäße, die Arteriolen, die in der Nasenscheidewand verlaufen, direkte Ableitungen der internen Halsschlagader sind und deshalb weniger von der Vasokonstriktion betroffen sind als die üblichen Messstellen wie beispielsweise am Finger, Zeh oder dem Ohrläppchen.

Anders ausgedrückt, ermöglichen Ausführungsbeispiele der vorliegenden Erfindung die Realisierung optischer Plethysmographie sowie der aus mehreren Wellenlängen abgeleiteten Blutsauerstoffsättigung, die in das Tragegestell einer Brille zur messtechnischen Anwendung auf dem Nasenrücken integriert ist. Dabei weist die Vorrichtung im Wesentlichen eine aktive, optische Sensoreinheit auf, und die Messung beruht auf dem Transmissionsprinzip und/oder dem Reflexionsprinzip. Noch anders ausgedrückt, stellen Ausführungsbeispiele der vorliegenden Erfindung einen tragbaren optischen Plethysmograph und ein optisches Pulsoximeter basierend auf dem Transmissionsprinzip und/oder dem Remissionsprinzip in Form einer Brille dar. Entsprechend können Ausführungsbeispiele auch als "Messvorrichtung zur Auswertung von Vitalparametern mittels optischer Transmissions- oder Reflexionsplethysmographie auf dem Nasenbein" oder "Verfahren zur Detektion von Vitalparametern mittels optischer Transmissions- oder Reflexionsplethysmographie auf dem Nasenbein" bezeichnet werden.

Das Anwendungsgebiet der Erfindung liegt im Bereich der präventiven, überwachenden und begleitenden Medizin für den Alltagseinsatz am Körper des Patienten.

Weitere Ausführungsbeispiele können auch als Vorrichtung zur Erfassung und Auswertung von Vitalparametern mittels optischer Plethysmographie auf dem Nasenrücken unter Verwendung transmissiver und/oder reflexiver Signalgewinnung bezeichnet werden, wobei die Vorrichtung einen Sensorkopf, in dem Lichtquellen und Lichtempfänger integriert sind und der auf dem Nasenrücken in das Tragegestell einer Brille integriert ist, aufweist, wobei räumlich getrennt die Ansteuer- und Auswerteelektronikeinheit, welche elektrisch mit dem Sensorkopf verbunden ist und durch eine Batterie bzw. einen Akkumulator mit Energie versorgt wird, angeordnet ist.

Weitere Ausführungsbeispiele dieser Vorrichtung haben die Form einer Brille und werten Vitalparameter mittels optischer Transmissions- oder Reflexionsplethysmographie aus.

Weiterbildungen dieser Ausführungsbeispiele weisen eine optimierte Treiberschaltung auf, die Leuchtdioden mit hoher Lichtintensität bzw. hohem Strom betreiben kann und dabei sehr kurze Lichtimpulse erzeugen kann, um die Signalqualität zu maximieren und den Stromverbrauch zu minimieren.

Noch weitergehende Weiterbildungen dieser Ausführungsbeispiele weisen einen Peak-Detektor auf, um eine zuverlässige digitale Abtastung des analogen plethysmographischen Signals zu ermöglichen.

Weitere Ausführungsbeispiele weisen eine Anordnung von Leuchtdioden und Photodioden als Sender und Empfänger auf, die über Multiplexer und Demultiplexer geschaltet bzw. ausgewählt werden können, um die Position der Messfläche bzw. des Messpunktes variieren zu können, um so mit möglichst nah an einer Arterie messen zu können und somit eine optimale Signalqualität auswählen bzw. erreichen zu können.

Darüber hinaus weisen Ausführungsbeispiele eine Auswahlvorrichtung auf, um die optimale Stelle, also eine Stelle möglichst nah an einer Arterie mittels Multiplexer und Demultiplexer und der Anordnung von Leuchtdioden und Photodioden zu finden und auszuwählen.

Abhängig von den Gegebenheiten können die Ausführungsbeispiele der erfindungsgemäßen Verfahren in Hardware oder in Software implementiert werden. Die Implementierung kann auf einem digitalen Speichermedium, insbesondere einer Diskette, CD oder DVD mit elektronisch auslesbaren Steuersignalen erfolgen, die so mit einem programmierbaren Computersystem zusammenwirken, das eines der Ausführungsbeispiele der erfindungsgemäßen Verfahren ausgeführt wird. Allgemein bestehen die Ausführungsbeispiele der vorliegenden Erfindung somit auch in Software-Programm-Produkten, bzw. Computer-Programm-Produkten bzw. Programm-Produkten mit einem auf einem Maschinen-lesbaren Träger gespeicherten Programmcode zur Durchführung eines der Ausführungsbeispiele der erfindungsgemäßen Verfahren, wenn eines der Software-Programm-Produkte auf einem Rechner oder auf einem Prozessor abläuft. In anderen Worten ausgedrückt, kann ein Ausführungsbeispiel der vorliegenden Erfindung somit als ein Computer-Programm bzw. Software-Programm bzw. Programm mit einem Programmcode zur Durchführung eines Ausführungsbeispiels eines erfindungsgemäßen Verfahrens realisiert werden, wenn das Programm auf einem Prozessor abläuft.

Der Prozessor kann hierbei von einem Computer, einer Chipkarte, einem digitalen Signalprozessor oder einem anderen integrierten Schaltkreis gebildet sein.

## Patentansprüche

1. Vorrichtung zum Erfassen zumindest eines Vitalparameters einer Person, mit folgenden Merkmalen:
einer optoelektronischen Sensoranordnung zum Erfassen des zumindest einen Vitalparameters mittels Lichttransmission, wobei die optoelektronische Sensoranordnung eine Lichtquelle (1) und ein lichtempfindliches Element (1') aufweist, wobei die Lichtquelle in einem ersten Seitenteil (110) eines Tragegestells (120) einer Brille angeordnet ist und das lichtempfindliche Element (1') in einem zweiten, dem ersten gegenüberliegenden Seitenteil (110') des Tragegestells (120) angeordnet ist.

2. Vorrichtung nach Anspruch 1, wobei das Tragegestell (120) der Brille starr ist und die Lichtquelle (1) und der Photosensor (1') eine durch das Tragegestell definierte feste geometrische Anordnung zueinander aufweisen.

3. Vorrichtung nach Anspruch 1 oder 2, wobei die Lichtquelle (1) in dem ersten Seitenteil (110) des Tragegestells (120) derart angeordnet ist, dass eine Richtung einer maximalen Lichtleistung (380) der Lichtquelle
(1) einem kürzesten Weg (380) des Lichts von der Lichtquelle (1) zu dem lichtempfindlichen Element (1') entspricht.

4. Vorrichtung nach einem der Ansprüche 1 bis 3, die ferner eine Steuereinrichtung (1004) aufweist,
wobei die optoelektronische Sensoranordnung eine erste Vielzahl (m) von Lichtquellen (1-16) aufweist, die in dem selben Wellenlängenbereich betrieben werden können, und eine zweite Vielzahl (n) von lichtempfindlichen Elementen (1'-16'),
wobei für die Transmissionsmessung die erste Vielzahl von Lichtquellen in dem ersten Seitenteil (110) des Tragegestells (120) angeordnet sind und die zweite Vielzahl von lichtempfindlichen Elementen in dem zweiten Seitenteil (110') des Tragegestells (120) angeordnet sind;
und die Steuereinrichtung (1004) ausgebildet ist, für die Erfassung des zumindest einen Vitalparameters eine Lichtquelle der ersten Vielzahl von Lichtquellen (1-16) und ein diesem zugeordnetes lichtempfindliches Element der zweiten Vielzahl der lichtempfindlichen Elemente (1'-16') auszuwählen.

5. Vorrichtung nach Anspruch 4, wobei die erste Vielzahl von Lichtquellen (1-16) eine erste Untermenge von Lichtquellen aufweist, die ausgebildet sind, um ein Licht eines ersten Wellenlängenbereichs zu erzeugen, und eine zweite Untermenge von Lichtquellen aufweist, die ausgebildet sind, um ein Licht eines zweiten von dem ersten unterschiedlichen Wellenlängenbereich zu erzeugen.

6. Vorrichtung zum Erfassen zumindest eines Vitalparameters einer Person mit folgenden Merkmalen:
einer optoelektronischen Sensoranordnung zum Erfassen des zumindest einen Vitalparameters mittels Lichtremission, wobei die optoelektronische Sensoranordnung eine Lichtquelle (1) und ein lichtempfindliches Element (1') aufweist, wobei die Lichtquelle (1) und das lichtempfindliche Element (1') in dem selben Seitenteil (110) des Tragegestells (120) der Brille angeordnet sind.

7. Vorrichtung nach Anspruch 6, die ferner eine Steuereinrichtung (1004) aufweist,
wobei die optoelektronische Sensoranordnung eine erste Vielzahl (m) von Lichtquellen (1-16) aufweist, die in dem selben Wellenlängenbereich betrieben werden können, und eine zweite Vielzahl (n) von lichtempfindlichen Elementen (1'-16'),
wobei für die Lichtremissionsmessung die erste Vielzahl von Lichtquellen und die zweite Vielzahl von lichtempfindlichen Elementen in dem selben Seitenteil (110) des Tragegestells (120) angeordnet sind;
und die Steuereinrichtung (1004) ausgebildet ist, für die Erfassung des zumindest einen Vitalparameters eine Lichtquelle der ersten Vielzahl von Lichtquellen (1-16) und ein diesem zugeordnetes lichtempfindliches Element der zweiten Vielzahl der lichtempfindlichen Elemente (1'-16') auszuwählen.

8. Vorrichtung nach Anspruch 7, wobei die erste Vielzahl von Lichtquellen (1-16) eine erste Untermenge von Lichtquellen aufweist, die ausgebildet sind, um ein Licht eines ersten Wellenlängenbereichs zu erzeugen, und eine zweite Untermenge von Lichtquellen aufweist, die ausgebildet sind, um ein Licht eines zweiten von dem ersten unterschiedlichen Wellenlängenbereich zu erzeugen.

9. Verfahren zum Erfassen zumindest eines Vitalparameters einer Person mittels einer optoelektronischen Sensoranordnung, wobei die optoelektronische Sensoranordnung eine erste Vielzahl (m) von Lichtquellen (1-16) und eine zweite Vielzahl (n) von lichtempfindlichen Elementen (1'-16') aufweist, wobei die erste Vielzahl von Lichtquellen in einem ersten Seitenteil (710) einer Brille angeordnet sind und die zweite Vielzahl von lichtempfindlichen Elementen in einem zweiten, den ersten gegenüberliegenden Seitenteil (710') der Brille angeordnet sind, wobei jeweils eine Lichtquelle der ersten Vielzahl von Lichtquellen einem lichtempfindlichen Element der zweiten Vielzahl der lichtempfindlichen Elementen zugeordnet ist und mit diesem eine auswählbare Messzuordnung einer dritten Vielzahl (1) von auswählbaren Messzuordnungen bildet,
wobei das Verfahren folgende Schritte aufweist:
Durchführen (810) zumindest einer optoelektronischen Messung für die auswählbaren Messzuordnungen, um jeweils zumindest ein Messergebnis zu erzeugen;
Bestimmen (820) einer amplitudenabhängigen Messgüte für jede der auswählbaren Messzuordnungen basierend auf dem jeweiligen zumindest einen Messergebnis;
Auswählen (830) der Messzuordnung (1-1') der dritten Vielzahl von Messzuordnungen mit der höchsten amplitudenabhängigen Messgüte; und
Durchführen (840) zumindest einer optoelektronischen Messung mittels der ausgewählten Messzuordnung (1-1'), um den zumindest einen Vitalparameter darauf basierend zu erfassen.

10. Verfahren nach Anspruch 9, mit folgenden weiteren Schritten:
fortlaufendes Bestimmen der amplitudenabhängigen Messgüte für die ausgewählte Messzuordnung;
Vergleichen der amplitudenabhängigen Messgüte mit einem Güteschwellwert; und
Auswählen einer anderen auswählbaren Messzuordnung der dritten Vielzahl (1) von Messzuordnungen oder erneutes Durchführen zumindest einer optoelektronischen Messung für jede der dritten Vielzahl von Messzuordnungen und Bestimmen einer Messgüte für jede der Vielzahl von Messzuordnungen, um erneut eine Auswahl der Messzuordnung mit der höchsten Messgüte durchzuführen, wenn die amplitudenabhängige Messgüte der momentan ausgewählten Messzuordnung kleiner als der Güteschwellwert ist.

11. Verfahren nach Anspruch 10 oder 11, mit folgenden Schritten:
nach dem Schritt des Bestimmens (820) einer amplitudenabhängigen Messgüte für jede der Messzuordnungen, Ordnen zumindest eines Teils der dritten Vielzahl von Messzuordnungen in einer Rangfolge gemäß ihrer amplitudenabhängigen Messgüte;
Auswahl (830) der Messzuordnung der Vielzahl von Messzuordnungen mit der höchsten amplitudenabhängigen Messgüte;
Durchführen (840) zumindest einer optoelektronischen Messung mittels der ausgewählten Messzuordnung (1-l'), um den zumindest einen Vitalparameter darauf basierend zu erfassen; und
Überwachen der Güte und Auswahl der Messzuordnung mit der nächst höchsten amplitudenabhängigen Messgüte gemäß der Rangfolge, wenn die amplitudenabhängigen Messgüte der momentan ausgewählten Messzuordnung kleiner als der Güteschwellwert ist.

12. Vitalparametererfassungssystem, mit folgenden Merkmalen:
einer Vorrichtung zum Erfassen zumindest eines Vitalparameters einer Person gemäß einem der Ansprüche 4,5,7 oder 8; und
einer Steuereinrichtung, die ausgebildet ist, ein Verfahren gemäß einem der Ansprüche 9 bis 11 auszuführen.
